# FASCICULE DE BREVET EUROPEEN

(11) **EP 1 933 747 B1**
(45) Date de publication et mention de la délivrance du brevet: **06.04.2011**
(21) Numéro de dépôt: 06793457.0
(22) Date de dépôt: 12.09.2006
(51) Int. Cl.: A61B 18/14, A61B 17/32

(54) **TETE DE DETECTION PER-OPERATOIRE APTE A ETRE COUPLEE A UN OUTIL D'EXERESE**
PEROPERATIVER FÜHLERKOPF ZUR VERBINDUNG MIT EINEM ABLATIONSINSTRUMENT
PEROPERATIVE SENSING HEAD ADAPTED TO BE COUPLED TO AN ABLATION TOOL

(30) Priorité: 13.09.2005 FR 0509329
(43) Date de publication de la demande: 25.06.2008
(73) Titulaire: Centre National de la Recherche Scientifique, 75016 Paris (FR)
(72) Inventeur: MENARD, Laurent, F-91120 Palaiseau (FR); BONZOM, Sébastien, F-94230 Cachan (FR); CHARON, Yves, F-91440 Bures sur Yvette (FR); DUVAL, Marie-Alix, F-91940 Les Ulis (FR); LEFEBVRE, Françoise, F-91120 Palaiseau (FR); PALFI, Stéphane, F-91530 Le Val Saint-Germain (FR); PINOT, Laurent, F-91510 Lardy (FR); SIEBERT, Rainer, F-78450 Villepreux (FR); PITRE, Stéphanie, F-92000 Nanterre (FR)
(74) Mandataire: Texier, Christian
(86) Numéro de dépôt international: PCT/EP2006/066287
(87) Numéro de publication internationale: WO 2007/031522

(56) Documents cités:
- US-A- 4 870 950
- US-A- 5 377 683
- US-A- 5 651 783
- US-A1- 2002 077 643

## Description

L'invention concerne l'assistance au traitement chirurgical des tissus biologiques, en particulier des tumeurs cancéreuses.

L'assistance au traitement chirurgical du cancer est aujourd'hui basée sur plusieurs techniques.

Selon un premier type de techniques dites « techniques d'imagerie pré-opératoire », le chirurgien réalise, avant l'opération, une image de la zone de tissus à traiter afin de localiser au mieux les parties de tissus à exciser.

La pré-localisation de la tumeur, à l'aide de la tomographie X ou de l'IRM permet, par exemple, d'obtenir la topographie anatomique précise du volume tumoral et de choisir ainsi les abords chirurgicaux les mieux adaptés. Couplé à un guidage stéréotaxique mécanique ou optique, le repérage pré-opératoire autorise des voies d'accès encore plus étroites et donc moins traumatisantes, notamment dans le cas de lésions profondes.

En neurochirurgie, cette technique peut être complétée par l'utilisation d'un appareil d'imagerie IRM fonctionnel, qui permet d'identifier précisément, avant l'intervention chirurgicale, les zones cérébrales fonctionnelles situées à proximité de la tumeur. Sur la base de cette information, le chirurgien peut ensuite optimiser l'étendue de la zone à exciser tout en minimisant les risques de morbidité post-opératoire.

Les techniques d'imagerie pré-opératoire ont permis de réaliser des gestes chirurgicaux plus précis et moins invasifs.

Toutefois, ces techniques présentent des limites en termes de performance et d'ergonomie. En particulier, ces techniques sont mal adaptées aux opérations nécessitant de localiser des tumeurs de petites tailles et leurs éventuelles disséminations métastatiques.

En outre, le déplacement des tissus au cours de l'intervention chirurgicale (en particulier dans le cerveau) rend souvent obsolète la localisation des lésions réalisée avant l'opération.

Selon un deuxième type de techniques, le chirurgien réalise, des prélèvements de tissus pendant l'opération et ces prélèvements sont analysés extemporanément, de manière à s'assurer de la qualité du geste opératoire du chirurgien.

Ces techniques qui s'appuient sur un diagnostic anatomopathologique précis des tissus prélevés présentent l'avantage d'être très fiables.

Cependant, ces techniques sont très coûteuses.

En outre, le temps nécessaire pour obtenir un diagnostic à partir des prélèvements peut parfois augmenter significativement la durée de l'intervention chirurgicale.

Compte tenu des inconvénients liés aux techniques d'imagerie pré-opératoire et aux techniques de prélèvements tissulaires, un troisième type de techniques dites « techniques per-opératoires » est apparu. Ces techniques utilisent des outils de contrôle capables de fonctionner en bloc opératoire et de suppléer ainsi les imageurs externes en aidant le praticien à définir plus précisément et en temps réel les marges d'une résection tumorale ou d'une biopsie.

Deux familles de techniques per-opératoires sont actuellement étudiées. La première famille de techniques, dites « techniques per-opératoires anatomiques », repose sur des systèmes d'imagerie anatomiques standard, tels que des systèmes d'endoscopie optique, d'échographie ultra-sonore, de tomographie X ou d'IRM. La deuxième famille de techniques, dites « techniques per-opératoires fonctionnelles », s'appuie sur la détection de signaux émis par les tissus grâce à des systèmes miniaturisés. Les signaux sont des particules ou des rayonnements émis par des traceurs radioactifs ou des molécules fluorescentes présents dans les tissus et spécifiques des lésions tumorales recherchées.

Selon les techniques per-opératoires anatomiques, le chirurgien utilise pour guider son geste un appareil d'imagerie anatomique de principe identique à ceux utilisés dans les services de diagnostic clinique mais dont les caractéristiques, en terme d'encombrement et d'ergonomie, ont été adaptées à l'utilisation en bloc opératoire.

Complémentaires des examens pré-opératoires, l'IRM bas-champ et la tomographie X sont principalement utilisés en bloc opératoire pour corriger les erreurs de localisation liées au déplacement des tissus pendant l'intervention et pour guider les procédures de biopsie. Les systèmes d'imagerie anatomique permettent en effet de renouveler en temps réel les images réalisées avant l'intervention et par conséquent de contrôler en temps réel la distorsion des structures anatomiques. L'évaluation de l'IRM per-opératoire pour la chirurgie des gliomes a ainsi montré que ces techniques permettent d'améliorer l'étendue de la résection tumorale par rapport aux interventions où seul le guidage stéréotaxique basé sur des images pré-opératoires était utilisé.

L'échographie ultra-sonore est également utilisée en bloc opératoire pour l'assistance au traitement chirurgical des tumeurs. Cette technique présente l'avantage d'être beaucoup moins lourde et coûteuse à mettre en place que l'IRM bas-champ ou la tomographie X. Le principal domaine d'application de l'échographie ultra-sonore per-opératoire est la localisation de tumeurs du sein non palpables et de tumeurs du foie. Plus généralement, cette technique est particulièrement adaptée à la localisation précise de lésions profondes.

Selon les techniques per-opératoires fonctionnelles, le chirurgien utilise un dispositif de détection miniaturisé capable de détecter des traceurs radioactifs ou des rayonnements lumineux spécifiques de l'histologie ou du comportement physiologique ou métabolique des lésions tumorales recherchées. La fonction d'un organe étant souvent altérée avant sa structure, ces techniques sont donc théoriquement plus sensibles et spécifiques que les techniques per-opératoires anatomiques pour différencier les tissus sains des tissus cancéreux.

Il est ainsi possible d'optimiser l'étendue de la résection tumorale au-delà des marges mises en évidence par les examens pré-opératoires et sans devoir attendre les résultats d'examens extemporanés de prélèvements tissulaires.

Ces techniques peuvent également être utilisées pour améliorer la précision du diagnostic des biopsies en guidant le chirurgien vers des régions tissulaires pertinentes pour déterminer la nature histologique de la tumeur.

De manière générale, la miniaturisation des dispositifs de détection utilisés permet également une utilisation plus aisée de la technique per-opératoire fonctionnelle en bloc opératoire car elle ne modifie que très peu le protocole chirurgical par rapport aux techniques per-opératoires anatomiques plus lourdes et contraignantes.

Pour assister efficacement le chirurgien dans la localisation des tissus tumoraux, le dispositif de détection miniaturisé doit présenter une résolution spatiale millimétrique compatible avec la précision du geste d'exérèse tumorale qui est de l'ordre de quelques millimètres cube (mm³). L'exérèse tumorale est réalisée par exemple par fragmentation des tissus biologiques au moyen d'un dispositif ultra-sonore.

En outre, le dispositif de détection doit présenter une sensibilité adaptée à la localisation de lésions de petites tailles (de l'ordre d'environ 1 mm³) pendant des temps d'acquisition adaptés à la pratique chirurgicale (typiquement inférieur à 10 secondes par mesure).

Le document US 5,651,783 décrit un ensemble à fibre optique destiné à recevoir un instrument chirurgical, tel qu'un instrument de chirurgie intraoculaire. L'ensemble comprend un manchon dans le corps duquel s'étend un faisceau de fibres optiques, et des moyens de connexion pour connecter le faisceau de fibres optiques contenu dans le manchon à un câble optique externe relié à une source de lumière. Le manchon à fibre optique permet la transmission de solution irrigante au site de traitement tout en transmettant de la lumière au point de chirurgie.

Un but l'invention est de permettre au chirurgien de réaliser une ablation avec une meilleure précision et une plus grande rapidité qu'avec les dispositifs de l'art antérieur.

Ce problème est résolu dans le cadre de la présente invention grâce à une sonde per-opératoire pour guider un outil d'exérèse, comprenant une tête de détection, ladite tête de détection comprenant :
- au moins une fibre optique apte à recevoir et guider un signal émis par une zone de tissus vers un équipement d'analyse,
- des moyens de fixation pour fixer la tête sur l'outil d'exérèse,
de sorte que l'outil d'exérèse soit apte à extraire une portion de tissus dans la zone de tissus émettant le signal.

La tête de détection est adaptée pour être couplée à un outil d'exérèse de sorte que le chirurgien peut réaliser en un seul geste, sans déplacer la sonde, les opérations de détection et d'ablation tumorale.

On obtient ainsi une localisation plus précise des tissus tumoraux puisqu'on élimine les erreurs de corrélation entre la position de la tumeur obtenue à partir du signal mesuré par la sonde et sa position réelle dans la plaie opératoire.

La possibilité de mesurer simultanément la concentration des traceurs radioactifs et des molécules fluorescentes permet en outre de bénéficier de la complémentarité des informations par ces deux méthodes et donc de renforcer la spécificité de la détection des tumeurs.

Avantageusement, une sonde selon l'invention mesurant un signal émis par des molécules fluorescentes dans une zone de tissus, en réponse à un signal lumineux d'excitation, mesure également un signal lumineux issu de la réflexion du signal lumineux d'excitation par les tissus. La spécificité est encore accrue.

Grâce à la tête de détection, qui enregistre les signaux émis par les tissus, le chirurgien peut observer en temps réel la zone de tissus traitée.

Avantageusement, on peut coupler la sonde à un système de neuronavigation pour permettre au chirurgien de visionner la position de la sonde par rapport à la tumeur et aux différentes structures cérébrales identifiées lors de l'IRM pré-opératoire.

En outre, la tête de détection peut facilement être remplacée par une tête de détection de caractéristiques différentes afin d'adapter la sonde aux contraintes spécifiques des différents protocoles chirurgicaux ainsi qu'aux différents signaux émis par les tissus.

La sonde est particulièrement adaptée à l'exérèse chirurgicale de tumeurs du système nerveux central, incluant le cerveau et la moelle épinière. En effet, la précision du traitement chirurgical de cette pathologie conditionne, plus que pour tout autre cancer, le pronostic vital et fonctionnel du patient.

La sonde peut présenter les caractéristiques des revendications 2 à 9.

L'invention se rapporte également à un outil de transmission et d'analyse pour une sonde per-opératoire conforme à la définition qui précède, l'outil étant selon la revendication 10.

L'outil peut présenter les caractéristiques des revendications 11 à 23.

D'autres caractéristiques et avantages ressortiront encore de la description qui suit, laquelle est purement illustrative et non limitative et doit être lue en regard des figures annexées parmi lesquelles :
- la figure 1 représente de manière schématique un ensemble de traitement chirurgical de tissus biologiques conforme à un mode de réalisation de l'invention,
- la figure 2 représente de manière schématique en vue frontale, une tête de détection d'une sonde per-opératoire conforme à un mode de réalisation de l'invention,
- la figure 3 représente de manière schématique en vue de côté et en coupe, une tête de détection d'une sonde per-opératoire conforme à un mode de réalisation de l'invention,
- la figure 4 représente de manière schématique en vue de côté et en coupe, une tête de détection d'une sonde per-opératoire conforme à une variante du mode de réalisation de la figure 3,
- les figures 5A et 5B représentent de manière schématique en vue de côté et en coupe, des organes de connexion destinés à permettre une connexion entre des fibres optiques de la tête de détection de la sonde et des fibres optiques de transmission vers des moyens d'analyse.

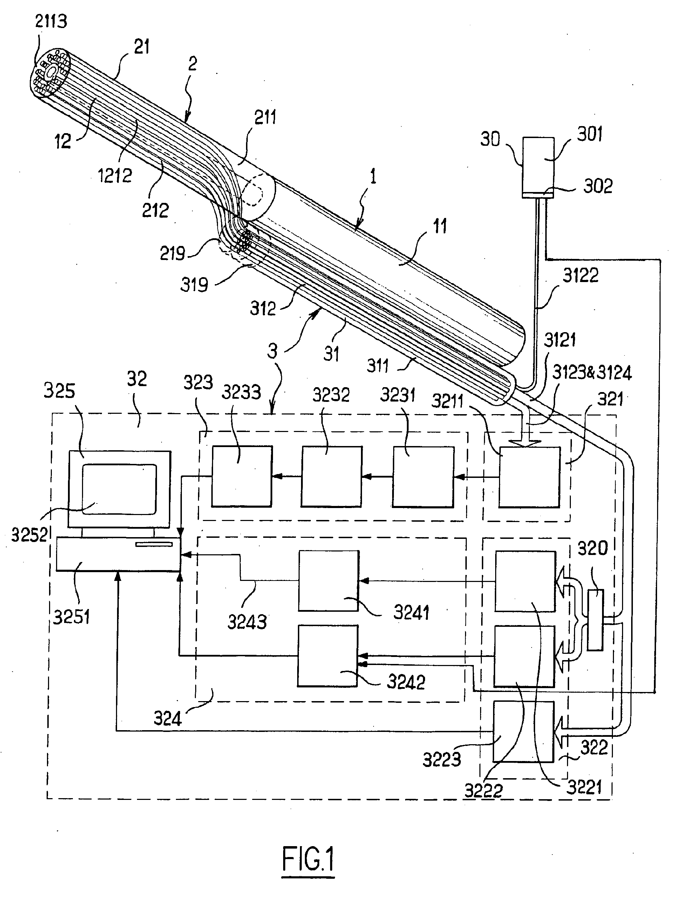

Sur la figure 1, l'ensemble de traitement chirurgical de tissus biologiques représenté comprend un outil d'exérèse 1, une sonde per-opératoire 2 et des moyens de transmission et d'analyse 3.

L'outil d'exérèse 1 comprend une partie de préhension 11 et une partie d'excision 12. L'outil d'exérèse 1 est par exemple un aspirateur ultra-sonore, notamment utilisé au cours du traitement chirurgical de gliomes pour exciser les tissus tumoraux. Dans le cas d'un aspirateur ultra-sonore, la partie d'excision 12 comprend un tube 1212 d'émission d'ultrasons et d'aspiration des tissus pulvérisés.

La sonde per-opératoire 2 comprend une tête de détection 21 formant une partie à usage unique. La tête de détection 21 se présente sous la forme d'un embout apte à être enchâssé sur l'outil d'exérèse 1.

La tête de détection 21 comprend un corps 211 de forme général cylindrique, un faisceau 212 de fibres optiques de détection s'étendant à l'intérieur du corps 211 et un organe de connexion 219.

Les moyens de transmission et d'analyse 3 comprennent une source lumineuse 30, un élément de transmission 31 réutilisable et un équipement d'analyse 32.

La source lumineuse 30 comprend un laser ou une lampe 301 et un filtre 302 d'excitation. Le laser ou la lampe 301 est apte à émettre de la lumière sous la forme d'un rayonnement continu ou d'impulsions lumineuses présentant une durée contrôlée. Le filtre 302 est apte à filtrer la lumière générée par le laser ou la lampe 301 pour transmettre un signal d'excitation contenant des photons présentant des longueurs d'ondes adaptées pour exciter des molécules fluorescentes contenues dans les tissus à traiter. Les molécules fluorescentes émettent alors un signal lumineux fluorescent, dont la longueur d'onde est différente de la longueur d'onde du signal d'excitation. La fluorescence est en effet un rayonnement électromagnétique, habituellement sous la forme de lumière visible ou infrarouge, provenant des molécules fluorescentes émettrices excitées par un signal lumineux d'excitation de plus courte longueur d'onde. Le rayonnement fluorescent cesse brusquement lorsque l'excitation cesse.

L'élément de transmission 31 comprend un corps 311, un faisceau 312 de fibres optiques de transmission s'étendant à l'intérieur du corps 311 et un organe de connexion 319. L'organe de connexion 319 est apte à coopérer avec l'organe de connexion 219 pour connecter chaque fibre optique de détection du faisceau 212 de la tête de détection 21 à une fibre optique de transmission du faisceau 312 de l'élément de transmission 31.

Comme illustré sur les figures 1 à 3, la tête de détection 21 comprend un corps 211 et un faisceau 212 de fibres optiques de détection s'étendant à l'intérieur du corps 211, dans une direction longitudinale de celui-ci.

Le corps 211 présente une forme générale tubulaire. Le corps 211 comprend une paroi 2111 cylindrique formée en métal, par exemple en acier inoxydable, ou en tout autre matériau compatible avec une intervention chirurgicale, et un canal central 2112. La paroi 2111 renferme le faisceau 212 de fibres optiques de détection. Les fibres optiques du faisceau 212 sont réparties autour du canal central 2112 et s'étendent sensiblement parallèlement au canal central 2112.

Le faisceau 212 de fibres optiques comprend une pluralité de fibres 2121 de détection de rayonnements lumineux, une fibre 2122 d'excitation, une pluralité de fibres 2123 de détection de traceurs radioactifs et une pluralité de fibres 2124 témoins.

Les fibres 2121, 2122 et 2123 s'étendent entre une surface 2113 d'extrémité du corps 211 et l'organe de connexion 219. Plus précisément, les extrémités des fibres 2121, 2122 et 2123 affleurent à la surface 2113.

Les fibres 2121 de détection de rayonnements lumineux sont constituées par des fibres claires. Ces fibres 2121 sont aptes à recevoir et guider un signal lumineux émis par des tissus biologiques.

La fibre 2122 d'excitation est également constituée par une fibre claire. Cette fibre 2122 est apte à guider un signal lumineux d'excitation généré par la source 30 en direction des tissus biologiques pour exciter des molécules fluorescentes contenues dans ces tissus.

Les fibres 2123 de détection de traceurs radioactifs comprennent une portion d'extrémité scintillante 2125 et une portion principale claire 2126, la portion d'extrémité scintillante 2125 étant fusionnée par exemple par chauffage à la portion principale claire 2126. La portion d'extrémité scintillante 2125 est apte à interagir avec des particules radioactives β (particules β⁺ ou particules (β⁻) émises par les tissus préalablement marqués par des traceurs radioactifs et à les convertir en signal lumineux. La portion principale 2126 est apte à guider le signal lumineux émis par la portion d'extrémité 2125.

La portion 2125 scintillante présente typiquement une longueur d'environ 1 mm et la portion 2126 claire présente typiquement une longueur d'environ 10 cm. Les portions scintillante 2125 et claire 2126 présentent typiquement un diamètre de l'ordre de 1,5 mm.

Les fibres témoins 2124 sont identiques aux fibres 2123 de détection de traceurs radioactifs, excepté que les fibres témoins 2124 sont rendues aveugles aux particules β. Plus précisément, les fibres témoins 2124 s'étendent en deçà de la surface 2113 d'extrémité du corps 211, de sorte que l'extrémité des fibres témoins 2124 est obstruée par une couche de métal d'épaisseur environ égale à 400 µm.

Les fibres 2123 de détection de traceurs radioactifs et les fibres témoins 2124 sont sensibles à des rayonnements γ de 511 électronvolt (eV) émis par les tissus après l'annihilation de particules β⁺. Ces rayonnements γ représentent un bruit de fond pour la détection de particules β⁺. Les fibres témoins 2124 permettent de quantifier le rayonnement γ en vue de le soustraire aux signaux mesurés par les fibres 2123 et d'obtenir ainsi un signal dû uniquement aux particules β⁺.

Pour permettre une quantification plus précise du rayonnement γ, le scintillateur plastique peut être remplacé par un scintillateur inorganique comme par exemple le lutétium oxyorthosilice dopé au cérium (LSO), qui a une densité plus élevée et donc une meilleure efficacité de détection pour le rayonnement γ.

La paroi 2111 dans laquelle les fibres optiques 2121, 2122, 2123 et 2124 du faisceau 212 de détection sont noyées constitue un blindage pour les fibres. Ce blindage isole les fibres de la lumière ambiante et de particules β parasites qui pourraient parvenir jusqu'aux portions scintillantes 2125 par les côtés ou par l'arrière des fibres 2123.

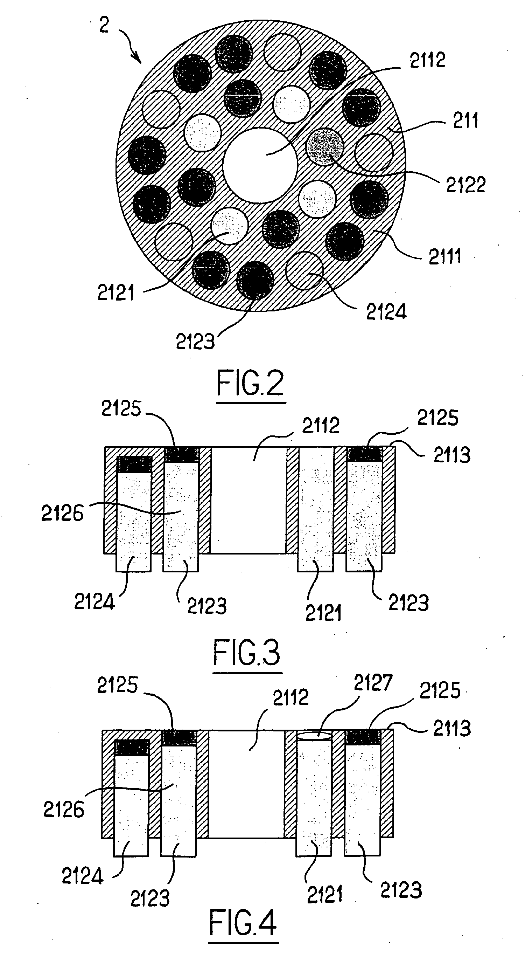

La figure 4 illustre une variante de la tête de détection 21. Dans cette variante, les fibres 2121 et 2122 de la tête de détection ne s'étendent pas jusqu'à la surface 2113 d'extrémité du corps 211. Plus précisément, les fibres 2121 et 2122 s'étendent en deçà de la surface 2113. La tête de détection 21 comprend une optique 2127 associée à chaque fibre 2121 et 2122, qui focalise la lumière issue des tissus vers les fibres 2121 et qui focalise la lumière issue de la fibre 2122 vers les tissus. Chaque optique 2127 comprend par exemple une microlentille. La focalisation de la lumière incidente permet d'augmenter la concentration lumineuse locale et par conséquent la sensibilité de l'ensemble de traitement. En outre, la collection de la lumière venant des tissus à l'aide d'une microlentille améliore la résolution spatiale de l'ensemble de traitement.

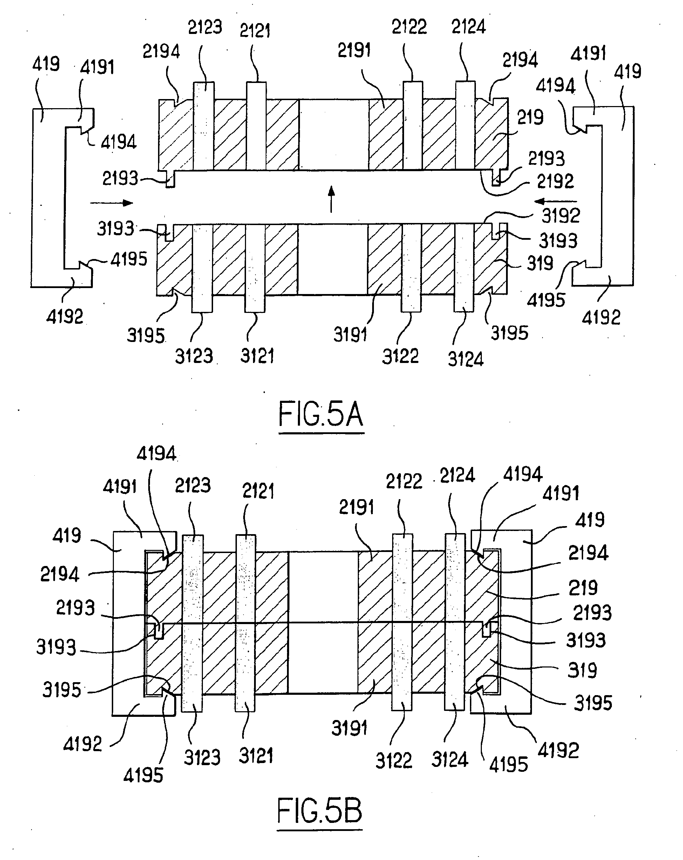

Les figures 5A et 5B représentent de manière schématique un exemple d'organes de connexion 219 et 319 destinés à permettre une connexion entre le faisceau 212 de fibres optiques de détection de la tête 21 de détection et le faisceau 312 de fibres optiques de transmission de l'élément 31 de transmission.

Sur la figure 5A les organes de connexion 219 et 319 sont détachés l'un de l'autre.

L'organe de connexion 219 comprend un corps 2191 dans lequel les fibres 2121, 2122, 2123 et 2124 de détection sont noyées. Le corps 2191 comprend une surface 2192 de connexion plane. Les extrémités des fibres 2121, 2122, 2123 et 2124 affleurent à la surface 2192 de connexion.

De la même manière, l'organe de connexion 319 comprend un corps 3191 dans lequel les fibres 3121, 3122, 3123 et 3124 de transmission sont noyées. Le corps 3191 comprend une surface 3192 de connexion plane. Les extrémités des fibres 3121, 3122, 3123 et 3124 affleurent à la surface 3192 de connexion.

Le corps 2191 comprend des pattes 2193 de connexion s'étendant en saillie à partir de la surface 2192 de connexion. Le corps 3191 comprend des orifices 3193 de connexion s'étendant à partir de la surface 3192 de connexion. Les pattes 2193 sont aptes à être introduites dans les orifices 3193 pour mettre en prise les organes de connexion 219 et 319. En outre, les pattes 2193 et les orifices 3193 sont agencés de sorte que lorsque les pattes 2193 sont introduites dans les orifices 3193, la surface 2192 de connexion vient en contact avec la surface 3192 de connexion et les extrémités des fibres 2121, 2122, 2123 et 2124 viennent en contact respectivement avec les extrémités des fibres 3121, 3122, 3123 et 3124 pour connecter les fibres entre elles.

L'ensemble de traitement chirurgical peut comprendre en outre des organes de verrouillage 419 destinés à maintenir les organes de connexion 219 et 319 en prise. Chaque organe de verrouillage présente une forme de U et comprend deux branches 4191 et 4192. Chaque branche 4191 et 4192 présente respectivement au niveau d'une extrémité libre un bossage 4194 et 4195.

Chacun des organes 219 et 319 de connexion comprend respectivement des encoches 2194 et 3195.

Les organes 419 de verrouillages sont aptes à venir enserrer les organes 219 et 319 de connexion lorsque ceux-ci sont en prise mutuelle. A cet effet, les organes 219 et 319 sont introduits entre les branches 4191 et 4192 des organes de verrouillage 419. La présence des bossages 4194 et 4195 provoque l'écartement des branches 4191 et 4192 par déformation élastique. Les bossages 4194 et 4195 sont ensuite aptes à être introduits dans les encoches 2194 et 3195 par retour élastique des branches 4191 et 4192.

Sur la figure 5B, les organes de connexion 219 et 319 sont mis en prise mutuelle et les organes de verrouillage 419 maintiennent les organes de connexion 219 et 319 en prise mutuelle.

Comme on le voit sur la figure 1, la tête de détection 21 à usage unique est apte à être fixée de manière détachable à l'outil d'exérèse 1. A cet effet, la partie d'excision 12 de l'outil 1 est apte à être insérée dans la tête de détection 21. Plus précisément, le tube d'aspiration 1212 de l'outil d'exérèse 1 est apte à être inséré dans le canal 2112 de la tête de détection 21 de sorte que l'extrémité aspirante du tube d'aspiration 1212 affleure à la surface d'extrémité 2113 de la tête de détection 21.

D'autre part, les organes de connexion 219 et 319 sont aptes à être mis en prise mutuellement pour coupler optiquement le faisceau 212 de fibres optiques de détection au faisceau 312 de fibres optiques de transmission. Les organes de connexion 219 et 319 sont des organes de connexion détachables, qui permettent une connexion et une déconnexion manuelle aisée.

La tête 21 de détection à usage unique peut donc être facilement remplacée par une autre tête.

L'élément de transmission 31 présente typiquement une longueur de 2 mètres pour acheminer les signaux lumineux issus de la tête de détection 21 vers l'équipement 32 d'analyse situé à l'extérieur du champ opératoire.

Lorsque les organes de connexion 219 et 319 sont mis en prise, les fibres 2121 de détection des rayonnements lumineux, la fibre d'excitation 2122, les fibres 2123 de détection des traceurs radioactifs et les fibres témoins 2124, du faisceau 212 de la tête 21 de détection sont respectivement connectées à des fibres de transmission 3121, 3122, 3123 et 3124, du faisceau 312 de l'élément 21 de transmission.

Les fibres 3121 de transmission sont connectées aux fibres claires 2121 de la tête de détection 21 et sont aptes à guider des rayonnements lumineux issus des tissus.

La fibre 3122 de transmission est connectée d'une part à la source 30 et d'autre part à la fibre d'excitation 2122 de manière à guider le rayonnement d'excitation émis par la source 30 jusqu'au tissus à traiter.

Les fibres 3123 et 3124 sont aptes à guider les signaux lumineux générés par l'interaction des particules β et des rayonnements γ émis par les tissus avec les portions scintillantes 2125 des fibres 2123 et 2124.

L'équipement d'analyse 32 comprend une première unité de photodétection 321, une deuxième unité de photodétection 322, une première unité d'acquisition 323, une deuxième unité d'acquisition 324 et un PC 325.

Les fibres 3123 et 3124 du faisceau 312 sont connectées à la première unité de photodétection 321.

La première unité de photodétection 321 comprend un photomultiplicateur multianode 3211. Le photomultiplicateur multianode 3211 comprend une pluralité de pixels (typiquement 64 pixels), chaque pixel ou groupe de pixels étant couplé à une fibre 3123 ou 3124 du faisceau 312. Chaque pixel du photomultiplicateur multianode est apte à convertir un signal lumineux qu'il reçoit en un signal électrique.

La première unité d'acquisition 323 comprend un réseau 3231 de résistances, une électronique de pré-amplification 3232 et une électronique d'amplification 3233.

Le réseau 3231 de résistances est apte à fournir selon le principe de la division de charge, une position du barycentre et une intensité du signal lumineux issus des fibres 3123 et 3124. Ces informations permettent de déterminer un numéro de fibre touchée et la quantité d'énergie cédée par une articule β ou un rayonnement γ aux portions scintillantes 2125 des fibres 2123 et 2124.

L'électronique de pré-amplification 3232 et l'électronique d'amplification 3233 sont aptes à intégrer puis à amplifier les signaux générés par le réseau 3231 de résistances. L'unité 323 transmet ensuite les signaux analogiques vers le PC 325 pour leur numérisation et leur traitement.

Par ailleurs, les fibres 3121 du faisceau 312 sont connectées à la deuxième unité de photodétection 322.

La deuxième unité de photodétection 322 comprend un spectrophotomètre fibré 3221, un détecteur 3222 ultra-rapide, par exemple un détecteur fourni par la société Hamamatsu Photonics sous la référence R3805U MCP PMT, et une photodiode à avalanche ou un photomultiplicateur multianode 3223 comprenant une électronique d'analyse intégrée, par exemple une photodiode fournie par la société id QUANTIQUE sous la référence id100-20. Le spectrophotomètre fibré 3221 est apte à détecter une intensité d'un signal lumineux qu'il reçoit en fonction d'une pluralité de longueurs d'ondes composant le signal lumineux et à convertir l'intensité en un signal électrique. Le détecteur 3222 ultra-rapide est apte mesurer un temps d'arrivée d'un signal lumineux qu'il reçoit et à convertir le temps d'arrivée en un signal électrique. La photodiode à avalanche ou le photomultiplicateur multianode 3223 est apte à convertir un signal lumineux qu'elle reçoit en un signal électrique standard.

La deuxième unité d'acquisition 324 comprend une première électronique de conversion 3241 et une deuxième électronique de routage 3242.

La première électronique de conversion 3241 est apte à convertir les signaux analogiques générés par le spectrophotomètre fibré 3221 en signaux numériques, pour permettre un transfert direct vers le PC 325 par un câble USB 3243.

La deuxième électronique de routage 3242 est apte à connecter plusieurs signaux en parallèle générés par un ou plusieurs détecteurs ultra-rapides 3222 à une carte de comptage de photons avec leur temps de passage.

Le PC 325 comprend une unité de numérisation et de calcul 3251 et un écran d'affichage 3252. L'unité de numérisation et de calcul 3251 est apte à recevoir et à traiter les signaux générés par les unités 323 et 324. L'unité de numérisation et de calcul 3251 est également apte à commander l'écran d'affichage 3252.

On va maintenant décrire l'utilisation et le fonctionnement de l'ensemble de traitement chirurgical qui vient d'être présenté.

Avant une opération, le chirurgien choisit une tête de détection 21 adaptée à la plaie opératoire et au type de tumeur à traiter.

Le chirurgien fixe la tête de détection 21 sur l'outil d'exérèse 1 en introduisant le tube d'aspiration 1212 dans le canal 2112 de la tête 21 de détection.

Puis, le chirurgien connecte les faisceaux 212 et 312 entre eux au moyen des organes de connexion 219 et 319.

Lors de l'opération, le chirurgien pratique une excision d'une partie principale visible de la tumeur.

Puis le chirurgien introduit l'extrémité de la sonde 2 dans la plaie opératoire. Plus précisément, le chirurgien positionne la sonde 2 de sorte que la surface d'extrémité 2113 de la tête 21 de détection soit positionnée en regard d'une zone des tissus à traiter. Le chirurgien parcourt la plaie opératoire à l'aide de la tête 21 de détection et positionne la tête de détection 21 en une pluralité de positions successives. Le chirurgien acquiert, pour chaque position de la tête 21, une cartographie des signaux émis de la zone des tissus en regard de la surface d'extrémité 2113. Pour chaque position de la tête 21 de détection, la durée d'acquisition de la cartographie n'excède pas quelques secondes.

La sonde per-opératoire permet tout d'abord de détecter des traceurs tumoraux radioactifs émetteurs de particules

Lorsqu'une particule β émise par la zone de tissus est reçue par l'une des fibres 2123, la portion scintillante 2125 de la fibre 2123 génère un signal lumineux (impulsion) qui est guidé par la portion claire 2126 de la fibre de détection 2123.

Le signal lumineux est guidé jusqu'à l'unité 321 de photodétection par une fibre de transmission 3123 de l'élément de transmission 31. Le signal lumineux est reçu par un pixel du photomultiplicateur 3211 qui génère un signal électrique (impulsion électrique) proportionnelle à l'intensité du signal lumineux.

Le signal électrique est traité par l'unité d'acquisition 323 qui fournit par l'intermédiaire du réseau de résistances 3231, un numéro associé à la fibre 2123 touchée par la particule β et une énergie cédée à la portion scintillante 2125. L'unité 323 d'acquisition amplifie et numérise les signaux générés par l'unité 321 de photodétection et transmet les signaux amplifiés à l'unité de numérisation et de calcul 3251.

Par ailleurs, les fibres de transmission 3124 guident le signal lumineux issu des fibres témoins 2124 sensibles uniquement aux rayonnements γ générés par les tissus.

Les rayonnements γ issus de l'annihilation des particules β⁺ dans les tissus représentent un bruit de fond pour le processus de localisation de lésions tumorales dans la plaie opératoire. Ces signaux peuvent en effet provenir de régions, spécifiques ou non spécifiques de fixation du traceur β⁺, très éloignées de la zone tissulaire analysée par la sonde.

Pour discriminer les signaux γ des signaux β⁺, l'ensemble de traitement chirurgical présente les caractéristiques suivantes.

Selon une première caractéristique, les portions 2125 scintillantes des fibres 2123 sont formées d'un matériau plastique, peu sensible aux rayonnements γ de haute énergie. Le matériau plastique présente en effet une faible densité (typiquement 1,05 g/cm³) et est constitué d'éléments possédant un faible numéro atomique (au maximum égal à 6 pour le Carbone). L'efficacité γ simulée d'une fibre plastique scintillante de 2 mm de diamètre et 1 mm de long placée à 0,1 mm d'une source ponctuelle de ¹⁸F est ainsi d'environ 300 coups par seconde par microCurie (cps/µCi) contre une efficacité β⁺ de 1,7.10⁴ cps/µCi dans la même configuration.

Dans le cas d'un traitement d'une tumeur cérébrale, ces rayonnements peuvent toutefois provenir de l'ensemble du cerveau. La contribution du bruit de fond γ au signal β⁺ peut donc devenir très importante en dépit de la faible sensibilité intrinsèque du matériau plastique constituant la portion scintillante.

Selon une deuxième caractéristique, l'unité 3251 de numérisation et de calcul est apte à sélectionner les signaux qu'elle reçoit en fonction de l'énergie de la particule ayant interagi avec la portion scintillante. L'étude théorique du spectre énergétique des rayonnements γ ayant interagi avec la portion scintillante montre en effet que 40% des rayonnements γ détectés génèrent une énergie comprise entre 0 et 100 keV, alors que la distribution énergétique des particules β⁺ est comprise entre 0 et 500 keV.

L'unité de numérisation et de calcul 3251 est donc apte à sélectionner uniquement les signaux dont l'énergie est supérieure à un seuil compris entre 50 et 100 kiloélectronvolt (keV).

Selon une troisième caractéristique, chaque fibre 2123 de détection de traceurs radioactifs est associée à une fibre témoin 2124. Les fibres témoins 2124 sont sensibles aux rayonnements γ mais sont insensibles aux particules β⁺. L'unité 3251 est apte à soustraire aux taux de comptage correspondant aux signaux générés par une fibre 2123 de détection de traceurs radioactifs, le taux de comptage γ correspondant aux signaux générés par la fibre témoin 2124 associée, afin d'obtenir uniquement une mesure du signal β⁺.

On notera à cet égard que plusieurs fibres 2123 de détection de traceurs radioactifs peuvent être associées à une même fibre témoin 2124 de manière à optimiser la portion de la surface de détection 2113 sensible aux particules β.

L'unité 3251 de numérisation et de calcul est apte à comparer le taux de comptage des impulsions correspondant à la concentration des traceurs radioactifs mesurés au niveau des fibres 2123 de la tête de détection 21 à un taux de comptage de référence préalablement mesuré dans une zone de la plaie opératoire constituée uniquement de tissus sains.

Lorsque la différence entre le taux de comptage mesuré et le taux de comptage de référence est supérieure à un seuil (par exemple écart-type supérieur à 3), l'unité 3251 identifiera la zone de tissus comme tumorale.

L'unité 3251 de numérisation et de calcul commande l'écran 3252 pour que l'écran affiche une cartographie de la zone de tissus sur laquelle est indiquée le nombre de particules β détectées par chaque fibre 2123.

Le chirurgien peut ainsi visualiser en temps réel sur l'écran 3252 une image de la zone tissulaire traitée qui indique la distribution de la concentration des traceurs radioactifs au niveau des fibres 2123 de la tête 21 de détection.

La sonde per-opératoire permet également la détection des tissus tumoraux par des techniques optiques fluorescentes.

Ainsi, les fibres claires 2121 reçoivent le rayonnement fluorescent émis par des molécules fluorescentes présentes dans les tissus après que les molécules ont été excitées par un signal lumineux d'excitation conduit par la fibre 2122 vers les tissus. Le signal lumineux contenant le rayonnement lumineux fluorescent est guidé par les fibres 2121 claires. Le signal lumineux est ensuite guidé via les fibres 3121 de transmission du faisceau 312 jusqu'à l'unité 322 de photodétection. Pour l'analyse du signal fluorescent, le signal lumineux est traité par un filtre 320 qui coupe les longueurs d'ondes du rayonnement d'excitation afin de ne sélectionner que le rayonnement fluorescent.

Le signal lumineux fluorescent est reçu d'une part par le spectrophotomètre fibré 3221 et d'autre part par le détecteur 3222 ultra-rapide. Le rayonnement fluorescent peut également être filtré puis reçu par la photodiode à avalanche ou le photomultiplicateur 3223. Les trois dispositifs 3221, 3222 et 3223 génèrent chacun un signal électrique.

Le spectrophotomètre fibré 3221 détermine un spectre du rayonnement fluorescent et génère un signal électrique permettant le comptage du rayonnement, une mesure de la longueur d'onde du rayonnement et un numéro identifiant la fibre qui a guidé le rayonnement.

La photodiode à avalanche ou le photomultiplicateur multianode 3223 génère un signal électrique qui est apte à réaliser le comptage des rayonnements fluorescents.

Le détecteur 3222 ultra-rapide génère un signal électrique correspondant au temps de passage des photons de fluorescence et un numéro identifiant la fibre qui a guidé ce photon. Ces signaux permettent une mesure du temps de décroissance du rayonnement de fluorescence par rapport au moment d'excitation des tissus (c'est à dire au moment d'émission d'une pulsation de la source lumineuse 30).

L'unité 324 d'acquisition numérise les signaux générés par le spectrophotomètre 3221 et transmet les signaux numérisés à l'unité de numérisation et de calcul 3251. Aussi, l'unité 3242 réalise un routage des signaux générés par le ou les détecteurs ultra-rapides 3222 et transmet les signaux vers la carte spécifique de numérisation de l'unité de numérisation et de calcul 3251. Les signaux générés par le détecteur 3223 sont transférés directement vers la carte de comptage de l'unité 3251.

L'unité de numérisation et de calcul 3251 est apte à comparer le taux de comptage des rayonnements détectés par les fibres 2121 à un taux de comptage de référence, préalablement mesuré dans une zone de la plaie opératoire constituée uniquement de tissus sains. Lorsque la différence entre ces taux de comptage est supérieure à un seuil prédéterminé (par exemple, le taux de comptage présente un écart-type supérieur à 3 par rapport au taux de comptage de référence), l'unité 3251 identifiera la zone de tissus comme tumorale.

En outre, l'unité 3251 de numérisation et de calcul est apte à comparer un spectre des rayonnements fluorescents détectés par les fibres 2121 à un spectre de référence préalablement mesuré dans une zone de la plaie opératoire constituée uniquement de tissus sains. La comparaison est réalisée pour une ou plusieurs gammes de longueurs d'ondes du spectre mesuré. Par exemple, lorsque la contribution « rouge » du spectre (longueurs d'ondes comprises entre 600 à 700 nm) est supérieure de plus de 100% par rapport au spectre de référence, l'unité 3251 identifiera la zone de tissus comme tumorale.

En outre, l'unité 3251 de numérisation et de calcul est apte à calculer les temps de décroissance de la florescence à partir des signaux de l'unité 3242 et à les comparer à des temps de référence préalablement mesurés dans une zone de la plaie opératoire constituée uniquement de tissus sains. Lorsque au moins un des temps de décroissance mesurés présente un écart supérieur de 50% par rapport au temps de référence, l'unité 3251 identifiera la zone de tissus comme tumorale.

Enfin, l'unité 3251 de numérisation et de calcul est apte à traiter en combinaison les différentes informations issues des dispositifs 3233, 3241, 3242 et 3223. L'identification d'un tissu tumoral est ainsi basée sur des informations complémentaires et conduisent à des résultats plus fiables qu'avec une utilisation indépendante des différentes informations.

En particulier, pour plusieurs mesures basées sur différents paramètres indépendants de la même zone de tissus indiquant une zone tumorale sans toutefois dépasser les seuils définis pour une zone de référence, l'unité 3251 identifiera la zone comme tumorale. Dans le cas contraire, où une mesure significative (écart-type supérieure à 3) n'est pas confirmée ou si elle est contredite par d'autres mesures, l'unité 3251 ne proposera pas l'excision des tissus correspondants.

L'unité de traitement 3251 commande l'écran 3252 pour que l'écran affiche plusieurs graphes bidimensionnels représentant chacun la zone de tissus. Sur le premier graphe sont indiquées le taux de comptage des tracteurs β au niveau de chaque fibre 2123.

L'unité 3251 peut commander l'écran 3252 pour présenter un deuxième graphe représentant l'intensité du rayonnement fluorescent en fonction de la longueur d'onde du rayonnement. Au préalable, le chirurgien aura choisi une fenêtre de longueur d'onde et l'écran 3251 n'affichera dans ce deuxième graphe que l'intensité du rayonnement fluorescent'au niveau des fibres 2121 dont les longueurs d'ondes sont comprises entre la limite inférieure et la limite supérieure de la fenêtre choisie par le chirurgien.

L'unité 3251 commande également l'écran 3252 pour que l'écran 3252 affiche un troisième graphe représentant le temps de décroissance des rayonnements émis par les molécules fluorescentes détectés au niveau des fibres 2121.

Le chirurgien peut ainsi visualiser en temps réel sur l'écran 3252 plusieurs cartographies de distributions spatiales d'intensités mesurées et des paramètres spécifiques de la zone tissulaire traitée.

L'écran 3252 peut afficher simultanément plusieurs cartographies mesurées et les superposer les unes aux autres.

L'écran 3252 peut également afficher un tableau récapitulant les résultats des mesures effectués individuellement et proposant au chirurgien des conclusions.

En fonction de la ou des cartographie(s) affichée(s) par l'écran 3252, le chirurgien peut décider d'exciser la zone de tissu à l'aide de l'outil d'exérèse 1.

On notera que le nombre et l'agencement des fibres optiques dans la tête de détection 21 peuvent aisément être modifiés pour être adaptés aux contraintes des différents protocoles chirurgicaux.

La sonde 2 peut être rendue polyvalente en prévoyant une gamme de têtes de détection 21 interchangeables répondant à différentes spécifications (compacité, sensibilité, résolution).

Il est ainsi possible de prévoir une tête de détection comprenant uniquement une fibre 2121 claire et une fibre 2122 d'excitation pour la détection des molécules fluorescentes présentes dans les tissus.

Il est également possible de prévoir une tête de détection comprenant uniquement une fibre 2123 de détection de traceurs radioactifs β et une fibre témoin 2124. Ce type de têtes de détection est particulièrement adapté aux opérations chirurgicales nécessitant une extrême compacité de la sonde, telles que des opérations d'exérèse sous endoscopie ou des biopsies.

A l'extrême inverse, il est possible de prévoir une tête de détection comprenant plusieurs couches concentriques de fibres de détection 2121 et 2123, permettant de réaliser une cartographie de la distribution spatiale des traceurs radioactifs et des rayonnements lumineux sur un champ de vue de l'ordre de 2 centimètres carré (cm²). Ce type de tête de détection est adapté aux opérations chirurgicales imposant une exploration rapide de la plaie opératoire ou aux opérations limitées par une fixation non spécifique importante des traceurs radioactifs.

Contrairement à une sonde de comptage, la possibilité de mesurer une cartographie de la zone de tissus à traiter permet en effet de discriminer les signaux tumoraux spécifiques du bruit de fond et donc de renforcer le rapport signal sur bruit qui peut être fortement dégradé par l'hétérogénéité de la fixation des traceurs dans les tissus environnant la lésion.

Entre ces deux configurations extrêmes qui viennent d'être exposées, des têtes de détections présentant des agencements de fibres intermédiaires en nombre et en positionnement peuvent également être prévues.

Avantageusement, il est possible de réunir et sommer les informations issues de chaque fibre en une seule information. On utilise alors la somme comme un seul détecteur mono-pixel, de telle manière que la sonde a une meilleure sensibilité pour une identification plus rapide des zones à traiter.

L'ensemble de traitement qui vient d'être décrit permet la mesure simultanée de la concentration de traceurs radioactifs et de la distribution des molécules fluorescentes. Cette association permet de bénéficier de la complémentarité des informations histologiques, métaboliques et moléculaires fournies par ces différentes mesures et ainsi de renforcer l'efficacité de la détection per-opératoire des tumeurs.

La sonde qui vient d'être décrite présente un encombrement réduit et une maniabilité permettant d'accéder à des régions étroites de plaie opératoire (cavités de l'ordre de 3 à 5 cm pour les tumeurs cérébrales).

En outre, la sonde permet un repérage précis et rapide des zones de tissus à extraire.

Le couplage avec l'outil d'exérèse permet en effet au chirurgien de visualiser en temps réel une cartographie de la zone de tissus se trouvant à proximité de l'outil d'exérèse et donc de réaliser en un seul geste, après un repérage, une excision plus précise et rapide des tissus tumoraux.

Enfin, la sonde permet une détection plus spécifique des tumeurs qu'avec les techniques de l'art antérieur. En effet, la combinaison d'une détection de plusieurs types de traceurs permet de fournir une information plus précise et fiable sur la nature des tissus traités.

Une sonde selon l'invention mesurant un signal émis par des molécules fluorescentes dans une zone de tissus, en réponse à un signal lumineux d'excitation, mesure avantageusement également un signal lumineux issu de la réflexion du signal lumineux d'excitation par les tissus.

Dans les développements qui précèdent, un faisceau de microfibres optiques de très faible diamètre peut bien entendu remplacer chaque fibre optique décrite.

## Revendications

1. Sonde (2) per-opératoire pour guider un outil d'exérèse, comprenant une tête (21) de détection, ladite tête de détection comprenant :
- au moins une fibre optique (2123) apte à recevoir et guider un signal émis par des traceurs radioactifs dans une zone de tissus, vers un équipement (32) d'analyse,
- des moyens (2112) de fixation pour fixer la tête (21) sur l'outil (1) d'exérèse,
de sorte que l'outil (1) d'exérèse soit apte à extraire une portion de tissus dans la zone de tissus émettant le signal, **caractérisée en ce que** la fibre optique (2123) comprenne une portion (2125) scintillante apte à interagir avec des particules radioactives émises par les tissus pour générer un signal lumineux

2. Sonde selon la revendication 1, dans laquelle la portion scintillante (2125) est apte à interagir avec une particule β émise par les tissus.

3. Sonde selon l'une des revendications 1 ou 2, dans laquelle la tête (21) de détection comprend en outre au moins une fibre (2124) témoin, insensible aux particules radioactives.

4. Sonde selon l'une des revendications qui précèdent, dans laquelle la tête (21) de détection comprend au moins une fibre optique claire (2121) apte à guider un signal lumineux émis ou réfléchi par les tissus.

5. Sonde selon la revendication 4. dans laquelle la tête (21) de détection comprend en outre des moyens optiques (2127) pour focaliser un signal émis ou réfléchi par les tissus vers la fibre optique claire (2121).

6. Sonde selon l'une des revendications qui précèdent, dans laquelle la tête (21) de détection comprend en outre au moins une fibre optique claire (2122) apte à acheminer vers les tissus un signal d'excitation pour que les tissus réfléchissent ledit signal ou émettent un signal fluorescent.

7. Sonde selon l'une des revendications qui précèdent, dans laquelle la tête (21) de détection comprend une pluralité de fibres (2121, 2122, 2123, 2124) agencées de sorte que, lorsque la tête (21) est fixée à l'outil (22) d'exérèse, les fibres (2121, 2122, 2123, 2124) sont réparties autour d'une partie d'excision (12) de l'outil (1) d'exérèse.

8. Sonde selon l'une des revendications qui précèdent, dans laquelle la tête de détection (21) comprend des moyens (219) de connexion associés à la fibre (2121, 2123) aptes à coopérer avec des moyens (319) de connexion complémentaires associés à un élément de transmission (31) pour acheminer des signaux émis par la zone de tissus vers un équipement (32) d'analyse des signaux.

9. Sonde selon l'une des revendications qui précèdent, dans laquelle la tête de détection (21) est à usage unique.

10. Ensemble de traitement chirurgical de tissus biologiques, comprenant au moins une sonde de détection selon l'une des revendications 1 à 9, et au moins un outil de transmission et d'analyse (3), l'outil comprenant :
- au moins une fibre optique (3121, 3123) de transmission apte à guider un signal issus de la tête de détection (21) de la sonde (2) vers un équipement d'analyse (32),
- des moyens (319) de connexion aptes à coopérer avec des moyens (219) de connexion complémentaires de la tête (21) de détection, pour connecter la fibre (3121, 3123) de transmission à une fibre (2121, 2123) de la tête (21) de détection.

11. Ensemble selon la revendication 10, dans lequel la fibre optique (3121, 3123) est apte à s'étendre le long d'une partie de préhension (11) de l'outil (1) d'exérèse lorsque la tête (21) de détection est fixée sur l'outil (1) d'exérèse.

12. Ensemble selon l'une des revendications 10 ou 11, comprenant une source (30) lumineuse d'excitation apte à être connectée à une fibre (2122) d'excitation de la tête (21) de détection pour transmettre un signal d'excitation vers les tissus.

13. Ensemble selon la revendication 12, comprenant en outre une fibre (3122) apte à acheminer un signal lumineux d'excitation depuis la source (30) jusqu'à une fibre (2122) d'excitation de la tête (21) de détection.

14. Ensemble selon l'une des revendications 12 ou 13, dans lequel la source (30) comprend un filtre (302) d'excitation apte à sélectionner une longueur d'onde ou une gamme de longueurs d'onde d'excitation spécifique(s).

15. Ensemble selon l'une des revendications 10 à 14, comprenant des moyens (321, 322) pour convertir un signal lumineux guidé par la fibre (3121, 3123) de transmission en signal électrique.

16. Ensemble selon la revendication 15, dans lequel les moyens (321) pour convertir le signal guidé par la fibre (3123) de transmission en signal électrique comprennent un photomultiplicateur multianode (3211) comprenant une pluralité de pixels, la fibre (3123) de transmission étant connectée à un pixel ou à un groupe de pixels apte à mesurer un nombre de particules radioactives ayant interagi avec une fibre (2123) de la tête (21) de détection et une position de la fibre (3123).

17. Ensemble selon l'une des revendications 15 ou 16, dans lequel les moyens (322) pour convertir le signal guidé par la fibre (3121) en signal électrique comprennent un photomultiplicateur ou une photodiode à avalanche (3223) apte à compter des impulsions du signal.

18. Ensemble selon l'une des revendications 15 à 17, dans lequel les moyens (322) pour convertir le signal guidé par la fibre (3121) en signal électrique comprennent un spectrophotomètre (3221) apte à mesurer une intensité d'un signal fluorescent émis par les tissus en fonction d'une longueur d'onde du signal fluorescent.

19. Ensemble selon l'une des revendications 15 à 18, dans lequel les moyens (321, 322) pour convertir le signal guidé par la fibre (3121) de transmission en signal électrique comprennent un détecteur (3222) apte à mesurer un temps d'arrivée par rapport à un signal d'excitation émis vers les tissus.

20. Ensemble selon l'une des revendications 10 à 19, comprenant en outre un filtre (320) apte à filtrer un signal lumineux guidé par une fibre (2121) de transmission pour éliminer une partie du signal lumineux résultant d'un rayonnement lumineux d'excitation réfléchi par les tissus et transmettre une partie du signal lumineux résultant d'un rayonnement fluorescent émis par les tissus.

21. Ensemble selon l'une des revendications 10 à 20, comprenant en outre des moyens de calcul (3251) aptes à traiter des signaux guidés par la tête (21) de détection et la fibre optique de transmission (3121, 3123) pour générer une cartographie de la zone de tissus.

22. Ensemble selon la revendication 21, dans lequel les moyens de calcul (3251) sont aptes à générer une cartographie à partir de signaux transmis par une fibre (2121, 2123, 2124) de la tête (21) de détection sensible à des particules radioactives émises par les tissus, à un signal lumineux réfléchi ou à un signal lumineux émis par les tissus.

23. Ensemble selon l'une des revendications 21 ou 22, comprenant des moyens d'affichage (3252), les moyens de calcul (3251) étant aptes à commander les moyens d'affichage (3252) pour qu'ils affichent une cartographie de la zone de tissus.

## Claims

1. A peroperative probe (2) for guiding an excision tool, comprising a detection head (21), said detection head comprising:
- at least one optical fibre (2123) for the reception and guidance of a signal, emitted by radioactive tracers in a tissue area, to an analysis equipment (32),
- attachment means (2112) for attaching the head (21) onto the excision tool (1),
so that the excision tool (1) is then adapted for removing a portion of tissue from the tissue area emitting the signal, **characterized in that** the optical fibre (2123) comprises a scintillating portion (2125) adapted to interact with radioactive particles emitted by the tissues for generating a light signal.

2. The probe according to claim 1, wherein the scintillating portion (2125) is adapted to interact with a P particle emitted by the tissues.

3. The probe according to any of claims 1 or 2, wherein the detection head (21) further comprises at least one control fibre (2124) which is insensitive to the radioactive particles.

4. The probe according to any of the preceding claims, wherein the detection head (21) comprises at least one clear optical fibre (2121) adapted to guide a light signal emitted or reflected by the tissues.

5. The probe according to claim 4, wherein the detection head (21) further comprises optical means (2127) to focus a signal emitted or reflected by the tissues to the clear optical fibre (2121).

6. The probe according to any of the preceding claims, wherein the detection head (21) further comprises at least one clear optical fibre (2122) adapted to route an excitation signal to the tissues so that the tissues will reflect said signal or emit a fluorescent signal.

7. The probe according to any of the preceding claims, wherein the detection head (21) comprises a plurality of fibres (2121, 2122, 2123, 2124) arranged so that, when the head (21) is attached to the excision tool (22), the fibres (2121, 2122, 2123, 2124) are distributed around an excision portion (12) of the excision tool (1).

8. The probe according to any of the preceding claims, wherein the detection head (21) comprises connection means (219) associated with the fibre (2121, 2123) adapted to mate with complementary connection means (319) associated with a transmission element(31) to route signals emitted by the tissue area to equipment (32) for analysis of the signals.

9. The probe according to any of the preceding claims, wherein the detection head (21) is for one-time use only.

10. An assembly for surgical treatment of biological tissues comprising at least one detection probe according to any of claims 1 to 9, and at least one transmission and analysis tool (3), the tool comprising:
- at least one optical transmission fibre (3121, 3123) adapted for guiding a signal obtained from the detection head (21) of the probe (2) to an analysis instrument (32),
- connection means (319) adapted for mating with complementary connection means (219) of the detection head (21), in order to connect the transmission fibre (3121, 3123) to a fibre (2121, 2123) of the detection head (21).

11. The assembly according to claim 10, wherein the optical fibre (3121, 3123) is adapted to extend along a gripping portion (11) of the excision tool (1) when the detection head (21) is attached onto the excision tool (1).

12. The assembly according to any of claims 10 or 11, comprising a light excitation source (30) adapted to be connected to an excitation fibre (2122) of the detection head (21) to transmit an excitation signal to the tissues.

13. The assembly according to claim 12, further comprising a fibre (3122) adapted to route a light excitation signal from the source (30) to an excitation fibre (2122) of the detection head (21).

14. The assembly according to any of claims 12 or 13, wherein the source (30) includes a filter (302) adapted to select a specific excitation wavelength or range of excitation wavelengths.

15. The assembly according to any of claims 10 to 14, comprising means (321, 322) for converting a light signal guided by the transmission fibre (3121, 3123) into an electrical signal.

16. The assembly according to claim 15, wherein the means (321) for converting the signal guided by the transmission fibre (3123) into an electrical signal, comprise a multi-anode photomultiplier (3211) comprising a plurality of pixels, the transmission fibre (3123) being connected to one pixel or a group of pixels, adapted to measure a number of radioactive particles that have interacted with a fibre (2123) of the detection head (21) and to a position of the fibre (3123).

17. The assembly according to any of claims 15 or 16, wherein the means (322) for converting the signal guided by the fibre (3121) into an electrical signal comprise a photomultiplier or an avalanche photodiode (3223) adapted to count the pulses of the signal.

18. The assembly according to any of claims 15 to 17, wherein the means (322) for converting the signal guided by the fibre (3121) into an electrical signal comprise a spectrophotometer (3221) adapted to measure an intensity of a fluorescent signal emitted by the tissues depending on a wavelength of the fluorescent signal.

19. The assembly according to any of claims 15 to 18, wherein the means (321, 322) for converting the signal guided by the fibre (3121) into an electrical signal comprise a detector (3222) adapted to measure an arrival time in relation to an excitation signal emitted to the tissues.

20. The assembly according to any of claims 10 to 19, further comprising a filter (320) adapted for filtering a light signal guided by a transmission fibre (2121) in order to eliminate a portion of the light signal resulting from light excitation radiation reflected by the tissues, and for transmitting a portion of the light signal resulting from fluorescent radiation emitted by the tissues.

21. The assembly according to any of claims 10 to 20, further comprising computation means (3251) adapted for processing signals guided by the detection head (21) and the transmission optical fibre (3121, 3123) to generate a mapping of the tissue area.

22. The assembly according to claim 21, wherein the computation means (3251) are adapted for generating a mapping from signals transmitted by a fibre (2121, 2123, 2124) of the detection head (21) which is sensitive to radioactive particles emitted by the tissues, to a reflected light signal or to a light signal emitted by the tissues.

23. The assembly according to any of claims 21 or 22, comprising display means (3252), the computation means (3251) being adapted for controlling the display means (3252) so that they display a mapping of the tissue area.

## Patentansprüche

1. Intraoperative Sonde (2) zur Führung eines Exhärese-Werkzeugs, umfassend einen Detektionskopf (21), wobei der Detektionskopf umfasst:
• mindestens einen Lichtleiter (2123) für die Aufnahme und Führung eines Signals, das von radioaktiven Tracern in einem Gewebebereich emittiert wird, in Richtung einer Auswerteeinrichtung (32),
• Befestigungsmittel (2112), um den Kopf (21) an dem Exhärese-Werkzeug (1) zu befestigen,
derart, dass das Exhärese-Werkzeug (1) imstande ist, in dem Gewebebereich, der das Signal emittiert, einen Gewebeteil zu entfernen,
**dadurch gekennzeichnet, dass** der Lichtleiter (2123) einen szintillierenden Teil (2125) aufweist, der imstande ist, mit radioaktiven Partikeln zu interagieren, die von dem Gewebe emittiert werden, um ein Lichtsignal zu erzeugen.

2. Sonde nach Anspruch 1, wobei der szintillierende Teil (2125) imstande ist, mit einem β-Partikel, das von dem Gewebe emittiert wird, zu interagieren.

3. Sonde nach einem der Ansprüche 1 oder 2, wobei der Detektionskopf (21) ferner mindestens einen Referenz-Lichtleiter (2124) umfasst, der gegenüber den radioaktiven Partikeln unempfindlich ist.

4. Sonde nach einem der vorhergehenden Ansprüche, wobei der Detektionskopf (21) mindestens einen klaren Lichtleiter (2121) umfasst, der für die Führung eines Lichtsignals, das von dem Gewebe emittiert oder reflektiert wird, vorgesehen ist.

5. Sonde nach Anspruch 4, wobei der Detektionskopf (21) ferner optische Mittel (2127) umfasst, um ein Signal, das von dem Gewebe emittiert oder reflektiert wird, in Richtung des klaren Lichtleiters (2121) zu fokussieren.

6. Sonde nach einem der vorhergehenden Ansprüche, wobei der Detektionskopf (21) ferner mindestens einen klaren Lichtleiter (2122) umfasst, der für die Weiterleitung eines
Erregungssignals in Richtung des Gewebes vorgesehen ist, derart, dass das Gewebe das Signal reflektiert oder ein fluoreszierendes Signal emittiert.

7. Sonde nach einem der vorhergehenden Ansprüche, wobei der Detektionskopf (21) eine Vielzahl von Lichtleitern (2121, 2122, 2123, 2124) umfasst, die so angeordnet sind, dass, wenn der Kopf (21) an dem Exhärese-Werkzeug (22) befestigt ist, die Lichtleiter (2121, 2122, 2123, 2124) um einen Exzisionsteil (12) des Exhärese-Werkzeugs (1) herum verteilt sind.

8. Sonde nach einem der vorhergehenden Ansprüche, wobei der Detektionskopf (21) Verbindungsmittel (219) umfasst, die dem Lichtleiter (2121, 2123) zugeordnet sind, für das Zusammenwirken mit komplementären Verbindungsmitteln (319), die einem Übertragungselement (31) zugeordnet sind, um Signale, die von dem Gewebebereich emittiert werden, in Richtung einer Einrichtung (32) zur Auswertung der Signale weiterzuleiten.

9. Sonde nach einem der vorhergehenden Ansprüche, wobei der Detektionskopf (21) zur einmaligen Verwendung vorgesehen ist.

10. Anordnung für die chirurgische Behandlung von biologischem Gewebe, umfassend mindestens eine Detektionssonde nach einem der Ansprüche 1 bis 9 und mindestens ein Werkzeug zur Übertragung und Auswertung (3), wobei das Werkzeug umfasst:
• mindestens einen Übertragungs-Lichtleiter (3121, 3123), der für die Führung eines Signals, das von dem Detektionskopf (21) der Sonde (2) ausgegeben wird, in Richtung einer Auswerteeinrichtung (32) vorgesehen ist,
• Verbindungsmittel (319), die für das Zusammenwirken mit komplementären Verbindungsmitteln (219) des Detektionskopfes (21) vorgesehen sind, um den Übertragungs-Lichtleiter (3121, 3123) mit einem Lichtleiter (2121, 2123) des Detektionskopfes (21) zu verbinden.

11. Anordnung nach Anspruch 10, wobei der Lichtleiter (3121, 3123) dafür vorgesehen ist, sich entlang eines Greifelements (11) des Exhärese-Werkzeugs (1) zu erstrecken, wenn der Detektionskopf (21) an dem Exhärese-Werkzeug (1) befestigt ist.

12. Anordnung nach einem der Ansprüche 10 oder 11, umfassend eine Erregungslichtquelle (30), die für die Verbindung mit einem Erregungs-Lichtleiter (2122) des Detektionskopfes (21) vorgesehen ist, um ein Erregungssignal in Richtung des Gewebes zu übertragen.

13. Anordnung nach Anspruch 12, ferner umfassend einen Lichtleiter (3122), der für die Weiterleitung eines Erregungslichtsignals von der Quelle (30) zu einem Erregungs-Lichtleiter (2122) des Detektionskopfes (21) vorgesehen ist.

14. Anordnung nach einem der Ansprüche 12 oder 13, wobei die Quelle (30) einen Erregungsfilter (302) umfasst, der für die Wahl einer spezifischen Erregungswellenlänge oder eines Erregungswellenlängenbereiches vorgesehen ist.

15. Anordnung nach einem der Ansprüche 10 bis 14, umfassend Mittel (321, 322) zur Umwandlung eines Lichtsignals, das von dem Übertragungs-Lichtleiter (3121, 3123) geführt wird, in ein elektrisches Signal.

16. Anordnung nach Anspruch 15, wobei die Mittel (321) zur Umwandlung des Signals, das von dem Übertragungs-Lichtleiter (3123) geführt wird, in ein elektrisches Signal einen Multianoden-Photoelektronenvervielfacher (3211) umfassen, der eine Vielzahl von Pixeln umfasst, wobei der Übertragungs-Lichtleiter (3123) mit einem Pixel oder einer Gruppe von Pixeln verbunden ist, das bzw. die für die Messung einer Anzahl von radioaktiven Partikeln vorgesehen ist, die mit einem Lichtleiter (2123) des Detektionskopfes (21) und einer Position des Lichtleiters (3123) zusammengewirkt haben.

17. Anordnung nach einem der Ansprüche 15 oder 16, wobei die Mittel (322) zur Umwandlung des Signals, das von dem Lichtleiter (3121) geführt wird, in ein elektrisches Signal einen Photoelektronenvervielfacher oder eine Lawinenphotodiode (3223) umfassen, der bzw. die für die Zählung der Signalimpulse vorgesehen ist.

18. Anordnung nach einem der Ansprüche 15 bis 17, wobei die Mittel (322) zur Umwandlung des Signals, das von dem Lichtleiter (3121) geführt wird, in ein elektrisches Signal ein Spektrophotometer (3221) umfassen, das für die Messung einer Stärke eines fluoreszierenden Signals, das von dem Gewebe emittiert wird, in Abhängigkeit von einer Wellenlänge des fluoreszierenden Signals vorgesehen ist.

19. Anordnung nach einem der Ansprüche 15 bis 18, wobei die Mittel (321, 322) zur Umwandlung des Signals, das von dem Übertragungs-Lichtleiter (3121) geführt wird, in ein elektrisches Signal einen Detektor (3222) umfassen, der für die Messung einer Ankunftszeit gegenüber einem Erregungssignal, das in Richtung des Gewebes emittiert wird, vorgesehen ist.

20. Anordnung nach einem der Ansprüche 10 bis 19, ferner umfassend einen Filter (320) für die Filterung eines Lichtsignals, das von einem Übertragungs-Lichtleiter (2121) geführt wird, um einen Teil des Lichtsignals, der aus einer Erregungslichtstrahlung, die von dem Gewebe reflektiert wird, resultiert, zu entfernen und einen Teil des Lichtsignals, der aus einer fluoreszierenden Strahlung, die von dem Gewebe emittiert wird, resultiert, zu übertragen.

21. Anordnung nach einem der Ansprüche 10 bis 20, ferner umfassend Rechenmittel (3251) für die Verarbeitung von Signalen, die von dem Detektionskopf (21) und dem Übertragungs-Lichtleiter (3121, 3123) geführt werden, um eine Kartographie des Gewebebereichs zu erzeugen.

22. Anordnung nach Anspruch 21, wobei die Rechenmittel (3251) vorgesehen sind für die Erzeugung einer Kartographie ausgehend von den Signalen, die von einem Lichtleiter (2121, 2123, 2124) des Detektionskopfes (21) übertragen werden, der gegenüber radioaktiven Partikeln, die von dem Gewebe emittiert werden, gegenüber einem reflektierten Lichtsignal oder gegenüber einem Lichtsignal, das von dem Gewebe emittiert wird, empfindlich ist.

23. Anordnung nach einem der Ansprüche 21 oder 22, umfassend Anzeigemittel (3252), wobei die Rechenmittel (3251) für die Steuerung der Anzeigemittel (3252) vorgesehen sind, derart, dass sie eine Kartographie des Gewebebereiches anzeigen.
